# EUROPÄISCHE PATENTSCHRIFT

(11) **EP 0 554 301 B1**
(45) Veröffentlichungstag und Bekanntmachung des Hinweises auf die Patenterteilung: **04.07.2001**
(21) Anmeldenummer: 91918300.4
(22) Anmeldetag: 21.10.1991
(51) Int. Cl.: A61L 27/00, A61F 2/30, A61F 2/28

(54) **IMPLANTAT UND VERFAHREN ZU SEINER HERSTELLUNG**
IMPLANT AND PROCESS FOR PRODUCING THE SAME
IMPLANT ET SON PROCEDE DE FABRICATION

(30) Priorität: 19.10.1990 DE 4033308
(43) Veröffentlichungstag der Anmeldung: 11.08.1993
(73) Patentinhaber: Draenert, Klaus, Dr.med.Dr.med.habil., D-81545 München (DE)
(72) Erfinder: Draenert, Klaus, Dr.med.Dr.med.habil., D-81545 München (DE)
(86) Internationale Anmeldenummer: EP9101999
(87) Internationale Veröffentlichungsnummer: WO9206718

(56) Entgegenhaltungen:
- WO-A-88/06023
- DE-A- 2 242 867
- DE-A- 3 445 709
- DE-A- 3 531 144
- US-A- 3 852 045

## Beschreibung

Die Erfindung betrifft ein Implantat, insbesondere einen Markraumstopper oder Markraumsperrer, und ein Verfahren zu seiner Herstellung.

Es besteht allgemein in der Medizin ein Bedürfnis nach Implantaten, die unter größtmöglicher Materialeinsparung und/oder bei niedrigem Gewicht ein Höchstmaß an Festigkeit bieten, sowie nach Implantaten mit durchgehender Porosität.

In der Hüftgelenkersatz-Chirurgie ist es zur Routine geworden, beispielsweise bei dem Ersatz des Hüftgelenkes, die präparierte Femurmarkhöhle, d.h. die Markhöhle des Oberschenkelknochens, mit einem selbsthärtenden Kunststoff, dem sogenannten Knochenzement, aufzufüllen und in der noch plastischen Masse die Metallkomponente des Prothesenschaftes zu verankern. In der klinischen Anwendung dieses Verfahrens zeigen sich sehr schnell zwei Probleme, nämlich zum einen, daß die plastische Zementmasse vom Prothesenschaft vor der Spitze der Prothesenkomponente in die Tiefe der Markhöhle vorgeschoben wird, und zum anderen, daß eine Querverpressung des Zementes nur dann erreicht werden kann, wenn dieses Absinken des Knochenzementes verhindert wird. Dies konnte durch Einführung eines Markraumstoppers oder Plugs (siehe z.B. Amstutz HC, Markolf KL, McNeice GM und Gruen TA, Proceedings of the fourth open scientific meeting of The Hip Society, Seiten 102 bis 116, St. Louis; Oh I, Carlson CE, Tomford WW und Harris WH, J. Bone Joint Surg 60A, Seiten 608 bis 613) zunächst gelöst werden. Ein weiteres Problem konnte erst nach sehr viel differenzierteren Untersuchungen in seiner Pathogenese aufgeklärt werden. Dieses war das Auftreten von ungeklärten Todesfällen nach Einbringen des Knochenzementes oder vor allem der Prothesenkomponente in den noch plastischen Knochenzement. Es konnte festgestellt werden, daß die sogenannte "high pressurizing"-Technik mit niedrigviskösen Knochenzementen diese Gefahr vergrößerte. In Tierexperimenten konnte der Zusammenhang zwischen der intramedulären Druckerhöhung und der tödlichen Lungenembolie gezeigt werden (Breed 1974, Experimental production of vascular high potention and bone marrow and fat embolism with methylmethacrylate cement. Traumatic high potention of bone. Clin Orthop 102:227-244). In Versuchen konnte danach nachgewiesen werden, daß das Einsaugen des Knochenzementes diese schwerwiegende und weit verbreitete Komplikation völlig vermeidet. Für die Vakuumapplikation ist es jedoch erforderlich - da hier über die Prothesenspitze über ein Bohrloch und eine Vakuumkanüle, welche eingeschraubt wurde, der Knochenzement in die Markhöhle eingesaugt wurde, vgl. z.B. PCT/EP/88/00122 - daß zwischen der Eröffnung der Vakuumkanüle, dem distal im Knochenrohr befindlichen Knochenmark und dem Knochenzement ein poröses Filter zwischengeschaltet wird. Im einfachsten Falle wird hierfür ein spongiöser Knochen, vorzugsweise ein kortikospongiöser Knochen dazwischengeschaltet, damit dieser für die Plazierung mechanisch ausreichend stabil ist. Bei vielen Operationen macht allerdings gerade die Gewinnung dieses zylinderförmigen Knochens Schwierigkeiten, so daß man bemüht war, einen durchgehend porösen, aber künstlichen Markraumstopper zu entwickeln.

Aus der DE-C2-31 06 917 ist ein Verfahren zur Herstellung eines Implantates als Knochenersatz in Form eines offenporigen bzw. offenzelligen Formkörpers aus körperverträglichem Metall bekannt, wobei das Metall unter Anwendung eines verlorenen Modells verarbeitet wird. Bei diesem Verfahren werden als Positivmodell offenporige bzw. offenzellige Natur- oder Kunstschwämme mit einer durchschnittlichen Weite der Poren oder Zellen zwischen 0,5 und 1,5 mm verwendet, die mit einer keramischen Einbettmasse gefüllt werden. Anschließend wird das Modellmaterial durch Hitze zerstört und entfernt, wodurch ein Keramik-Negativmodell entsteht. Danach werden die zuvor von dem Material des Positivmodells, d.h. dem Natur- oder Kunstschwamm, eingenommenen Räume durch ein gieß- oder schleuderbares Metall gefüllt und anschließend wird das Keramikmaterial des Negativmodells wieder entfernt. Ein Nachteil dieses Verfahrens besteht darin, daß die Struktur des Positivmodells und damit die Struktur des fertigen Metallimplantates nicht gezielt einstellbar ist, sondern daß die Struktur jeweils so akzeptiert werden muß, wie die Schwämme, seien es Natur- oder Kunstschwämme, beschaffen sind. Ein weiterer Nachteil dieses Verfahrens besteht darin, daß es umständlich ist und zwei Formkörper, d.h. zunächst ein Positivmodell und danach ein Negativmodell, benötigt werden, um letztlich ein dreidimensionales Gerüst zu schaffen.

Der Erfindung liegt die Aufgabe zugrunde, ein Implantat, insbesondere einen Markraumsperrer, und ein Verfahren zu seiner Herstellung bereitzustellen, das sowohl im Hinblick auf seine Porosität als auch im Hinblick auf die Formstabilität und andere gewünschte Eigenschaften, wie Löslichkeit oder Resorbierbarkeit, exakt nach den jeweiligen Bedürfnissen einstellbar ist.

Diese Aufgabe wird durch die Erfindung gemäß den Patentansprüchen gelöst.

Die Erfindung geht von dem Grundgedanken aus, miteinander verbundene einzelne Formkörper, z.B. in Form einer dichten Packung oder eines Konglomerats, als Platzhalter für die poröse Hohlraumstruktur des Implantats zu verwenden und um die leicht entfernbaren, beispielsweise leicht löslichen oder leicht schmelzbaren Formkörper herum ein dreidimensionales Gerüst zu schaffen, vorzugsweise aus einem gießfähigen Material. Im Anschluß daran können die Formkörper -entweder physikalisch oder chemisch, vorzugsweise durch Anwendung des Prinzips der Wasserlöslichkeit oder der Schmelzbarkeit, oder durch Anwendung von Hitze wieder entfernt werden. Dabei wird vorzugsweise jeweils von einem Negativmodell des fertigen Implantats ausgegangen.

Anders ausgedrückt, beruht die Erfindung im Prinzip darauf, daß durch wasserlösliche oder schmelzbare Formkörper vorgegebener Konfiguration, beispielsweise kugelförmige Formkörper oder Formkörper in Form eines Granulates, welche punktförmig miteinander zu einer dreidimensionalen Struktur in Form eines Konglomerats verschweißt werden können, beispielsweise durch Wasserdampf, eine durchgehende bälkchenförmige oder trabekuläre Hohlraumstruktur entsteht, die mit einem anderen Material, beispielsweise Kunststoff oder einem Verbundmaterial aus einem Kunststoff in Verbindung mit Füllerpartikeln, ausgefüllt werden kann. Dieses Ausfüllen erfolgt vorzugsweise im Gießverfahren oder unter Anwendung von Spritzgußverfahren. Die räumliche Struktur des zum Ausfüllen verwendeten Materials stellt das "Positivmodell" zu dem von den Formkörpern gebildeten "Negativmodell" dar und ist durch geeignete Wahl der Struktur des Negativmodells beliebig einstellbar. Der so entstandene Verbundimplantatkörper kann anschließend von den ein "Inlet" bildenden Formkörpern befreit werden. Wenn die Formkörper wasserlöslich sind, kann dies einfach und billig durch Einbringen des Verbundkörpers in Wasser und gegebenenfalls Erzeugen einer Strömung oder Turbulenz, beispielsweise in einer Waschmaschine oder einer Drehtrommel durchgeführt werden. Wenn die Formkörper leicht schmelzbar sind, kann die Entfernung durch Einwirkung von Hitze durchgeführt werden. Durch die Entfernung der Formkörper entsteht ein durchgehend poröser Implantatkörper. Die Porosität des Implantatkörpers ist durch geeignete Auswahl der Größe, Größenverteilung und Schüttdichte der Formkörper sowie deren Verschweißung miteinander reproduzierbar einstellbar. Das Implantat ist auch mechanisch widerstandsfähig und kann in seiner äußeren Form beliebig gestaltet werden.

Die Porosität des Implantatkörpers wird so eingestellt, daß zwar Flüssigkeiten, wie beispielsweise Blut, durch Anlegen eines gewissen Unterdrucks durch den Implantatkörper gesaugt werden können, daß aber andererseits hochvisköse Flüssigkeiten, Knochenzement und kleine Partikel, wie Debrispartikel, Knochenbälkchen, Knochenmark und geronnenes Blut im Implantatkörper hängenbleiben. Das Implantat wirkt somit als Filtersystem.

Entlang der gegebenenfalls beschichteten Oberflächen der vorgebbar einstellbaren Hohlraumstruktur des Implantats kann es erwiesenermaßen sehr schnell zu einem gezielten Knocheneinwuchs kommen, der das gesamte Implantat auch unabhängig von der biomechanischen Beanspruchung knöchern durchwachsen kann.

Das erfindungsgemäße Implantat kann auch als Wirkstoffträger in sogenannten "drug delivery"-Systemen Verwendung finden. Das erfindungsgemäße Implantat kann auch als Träger für alle diejenigen Wirkstoffe verwendet werden, die auf Oberflächen aufgetragen werden können und zu denen das "bone morphogenetic protein" oder gewisse Wachstumsfaktoren zählen.

Im Prinzip können alle beliebigen Werkstoffe verwendet werden, indem beispielsweise wasserlösliche oder säurelösliche Formkörper mit schmelzbaren Materialien oder wasserlösliche oder säurelösliche Formkörper mit sinterfähigen, gießbaren Materialverbunden oder im Spritzgußverfahren verarbeitbaren Kunststoffen oder gießbaren, beispielsweise im Schleudergußoder Spritzguß verarbeitbare Metallen, Metallegierungen oder Metallverbundwerkstoffen in der Weise kombiniert werden, daß jeweils eine Sorte Formkörpert, entweder physikalisch oder chemisch oder auf andere Weise, wieder herauslösbar ist und das dreidimensionale Gerüst als Stützgerüst verbleibt. Das Stützgerüst kann anschließend durch physikalische oder chemische Verfahren verfestigt, oberflächenbehandelt oder mechanisch nachbearbeitet werden.

Die Formkörper und das aus einem gießbaren Material bestehende Stützgerüst können auch zusammen im Verbund weiterverarbeitet werden, mechanisch nachbearbeitet, und erst im Anschluß daran physikalisch oder chemisch separiert werden.

Das Material für das dreidimensionale Gerüst ist vorzugsweise gießbar oder spritzbar, beispielsweise im Spritzgußverfahren. Vorzugsweise besteht das dreidimensionale Gerüst des erfindungsgemäßen Implantates aus einem Polymer auf der Basis eines Polyacrylates oder eines Polymethacrylates, einem Copolymer eines Acrylates und Methacrylates, einer Mischung aus diesen oder aus einem anderen körperverträglichen Kunststoff. Das dreidimensionale Gerüst kann auch aus einer resorbierbaren Polyaminosäure, einem Polylactat, einem Polyglykolat, einem Gemisch aus verschiedenen Polyaminosäuren oder einem anderen im Körper auflösbaren und/oder resorbierbaren Material bestehen.

Als Material für das dreidimensionale Gerüst des Implantats wird vorzugsweise Material verwendet, das auch als Knochenzement verwendet wird, da zum einen die Bioverträglichkeit dieses Materials nachgewiesen ist und sich zum anderen dieses Material in idealer Weise mit der Knochenzementscheide als solche verbindet.

Die als Platzhalter für das Hohlraumsystem des Werkstoffes dienenden Formkörper weisen vorzugsweise die Form von Kugeln oder gleichmäßigen geometrischen Körpern, beispielsweise Vielecken auf, es kann aber auch Granulatmaterial als Formkörper verwendet werden. Vorzugsweise ist das Material der Formkörper leicht löslich, z.B. wasserlöslich oder säurelöslich, oder leicht schmelzbar. Besonders bevorzugt sind Formkörper aus einem wasserlöslichen Material, beispielsweise aus Zucker, die zunächst im Wasserdampf zur Bildung eines Formkörperkonglomerats fest miteinander verklebt werden können und nach der Ausbildung und Aushärtung des dreidimensionalen Gerüsts im Wasserbad oder in einer Waschmaschine ausgewaschen werden können, oder Formkörper aus einem leicht schmelzbaren Material, wie Wachs.

Als Material für die Formkörper kann auch ein anorganischer oder keramischer Werkstoff. Wie Tricalciumphosphat, ein Hydroxylapatit, ein Gemisch aus beiden oder eine andere, leicht oder schwer resorbierbare Calciumverbindung verwendet werden, wobei die keramischen Werkstoffe in der Regel durch Säure aus dem Verbund herausgelöst werden können.

Da sowohl die Größe als auch die Schüttdichte und die Art der gegenseitigen Verbindung der als Platzhalter für das Hohlraumsystem des Implantats dienenden Formkörper frei wählbar ist, ist auch das Hohlraumsystem des fertigen Implantates bezüglich seiner Porosität frei einstellbar. Beispielsweise können kugelförmige Formkörper verwendet werden, wenn ein Hohlraumsystem mit im wesentlichen kugelförmigen und miteinander in Verbindung stehenden Hohlräumen angestrebt wird. Wenn ein Hohlraumsystem mit unterschiedlich großen Hohlräumen angestrebt wird, können Formkörper unterschiedlicher Größe und/oder Form miteinander gemischt werden. Die gesamte Porosität des fertigen Implantats ist durch Einstellung, Variation und Kombination der Form und/oder der Schüttdichte der Formkörper und/oder Wahl des Verfahrens, durch das die Formkörper zu einem Konglomerat miteinander verbunden werden, gezielt einstellbar. Die Form und Gestaltung der Bälkchen bzw. Trabekel des dreidimensionalen Gerüsts ist erfindungsgemäß ebenso vorgebbar und gezielt je nach Verwendungszweck einstellbar.

Die Formkörper können auch durch ein Sinterverfahren miteinander verbunden werden. Die bevorzugte Größe der Formkörper beträgt zwischen 0,2 und 5 mm, vorzugsweise bis 3 mm, besonders bevorzugt 1 bis 1,5 mm.

Eine weitere Variation der gewünschten Eigenschaften des fertigen Implantates ist auch dadurch möglich, daß das dreidimensionale Gerüst zusammen mit den Formkörpern verdichtet wird, beispielsweise im HIP-Verfahren (high isostatic pressure-Verfahren).

Das Implantat kann auch zusätzlich Füllerpartikel enthalten, beispielsweise Tricalciumphosphat oder Hydroxylapatit oder ein Gemisch aus beiden, mit einem Anteil von 1 bis 95 %, vorzugsweise bis 80 %, und mit einer Partikelgröße bis 300 *µ*m, vorzugsweise bis zu 250 *µ*m und einem Porenvolumen von 0,1 ml/g bis 0,8 ml/g.

Je nach der beabsichtigten Verwendung können dem Implantat auch verschiedene Wirkstoffe zugesetzt werden, beispielsweise kann das Material für das dreidimensionale Gerüst 0,01 bis 10 % eines Wirkstoffes enthalten, der aus dem Implantat verzögert freigesetzt werden kann. Als Wirkstoffe kommen Antibiotika, ein das Knochenwachstum induzierender Wirkstoff, ein Wachstumsfaktor oder ein anderer chemotaktisch oder hormonell wirkender Faktor in Betracht, der das Einsprossen der Gefäße oder direkt die Stimulation der Osteoblasten bewirkt. Es können auch Zytostatika, wie Methotrexat, zugesetzt werden.

Die äußere Form des Implantats ist frei wählbar und richtet sich nach dem jeweiligen Verwendungszweck. Wenn das erfindungsgemäße Implantat als Markraumstopper oder Markraumsperrer verwendet wird, wird seine äußere Form so gestaltet, daß es ohne Schwierigkeiten in die Markhöhle bis etwa 2 cm unterhalb der für die Prothesenspitze vorgesehenen Position plaziert werden kann. Im Anschluß daran wird der Markraumsperrer über einen Knochenbohrer angebohrt und mit einer Vakuumkanüle verbunden, wie beispielsweise in der WO 88/06023 beschrieben. Auf diese Weise wird der Knochenzement beim Einbringen in die Markhöhle in der als Filtersystem dienenden porösen Struktur des Implantats gestoppt und die Saugkanüle bleibt frei von Knochenzement. Andererseits können Flüssigkeiten über die Vakuumkanüle abgesaugt werden, so daß der Knochenzement in ein bluttrockenes Bett eingesaugt wird, was erhebliche Vorteile mit sich bringt. Es wird durch das poröse Filtersystem des Implantats auch verhindert, daß Debrispartikel, Knochenbälkchen, Knochenmark und geronnenes Blut die Saugkanüle verstopfen, da diese Partikel im Filtersystem des Implantats hängen bleiben.

Das erfindungsgemäß als Markraumsperrer dienende Implantat ist vorzugsweise kegelstumpfförmig mit einer Konizität von vorzugsweise weniger als 10°, besonders bevorzugt etwa 2° bis 5°, oder zylinderförmig ausgebildet, wobei die äußeren Abmessungen an die Abmessungen des Markraums angepaßt sind. Zur Verbesserung seiner Verankerung und zur Verbesserung der Abdichtung weist der Markraumsperrer außen im Abstand von 0,5 bis 3,0 mm in Axialrichtung mehrere Lamellen mit einer Lamellendicke von 0,3 mm bis 1,0 mm auf. Die Lamellen können entweder gleichförmig ringförmig um den Zylinder angeordnet sein, oder die Form eines Gewindes aufweisen. Das Gewinde erstreckt sich spiralförmig mit einer Steigung, die zwischen 30 und 87° variiert.

Der Markraumsperrer so ausgebildet, daß verhindert werden kann, daß z.B. bei einer Vakuumapplikation von Knochenzement von der distalen Hälfte des Femurmarkkanales in unerwünschter Weise Knochenmark und Blut über eine angelegte Vakuumkanüle abgesaugt wird. Dies kann dadurch realisiert werden, daß der Markraumsperrer an seinem Boden verschlossen ist, in Form eines Deckels oder einer Platte mit einer Dicke von 0,5 bis 2 mm. Dieser Deckel kann entweder mechanisch ausgebildet sein, oder er kann durch geeignete Maßnahmen bei der Herstellung des Implantats als geschlossene Oberflächenschicht gebildet werden. Hierbei kann beispielsweise die Auffüllung mit den vorzugsweise kugelförmigen Formkörpern zunächst ausgespart bleiben, so daß sich die Bodenplatte vollständig mit dem für die Bälkchenstruktur verwendeten Material, beispielsweise Kunststoff auffüllt. Der Deckel kann verschiedene Form aufweisen, er kann gerade oder abgerundet sein oder in Richtung auf die Tiefe der Markhöhle einen zapfenförmigen Abschluß aufweisen. Das Dach des Markraumsperrers ist dagegen porös.

Wenn das erfindungsgemäße Implantat als Verschluß eines Amputationsstumpfes ausgebildet ist, ist seine äußere Form zylindrisch, gegebenenfalls mit einem breiteren, halbkugelförmigen oder konvexen Abschluß, der nach der Implantation das zu verschließende freie Knochenende überragt und abdichtet. Vorteilhafterweise wird die Porosität des Verschlusses im Bereich seines distalen Endes vollständig abgedichtet. Dies kann dadurch erfolgen, daß die Form im Spritzgußwerkzeug die Dicke des dicht auszubildenden Bereichs in seiner Gesamthöhe freiläßt und eine Auffüllung mit den löslichen Formkörpern nur bis zu dieser Grenzmarke erfolgt. Die Düse, über die der das dreidimensionale Gerüst des Implantats bildende Werkstoff, vorzugsweise ein Kunststoff, eingespritzt wird, befindet sich dabei auf der Seite des dicht auszubildenden Bereichs. Dies bewirkt, daß die Formkörper durch den Druck angehoben werden und sich am distalen Ende des Verschlusses eine geschlossene Platte aus dem Werkstoff ausbildet, die einen Deckel bildet. Diese Platte weist eine Dicke von 0,5 bis 2 mm, besonders bevorzugt bis 1 mm auf. Das proximale Ende des Amputationsstumpf-Verschlusses ist dagegen porös.

Die äußere Form des Implantates wird derart gewählt, daß sie für den Zweck als Markraumsperrer oder für den Zweck als Markhöhlenverschluß, insbesondere für Amputationsstümpfe, geeignet ist, wie dies beispielsweise in der WO 86/03667 vorgeschlagen ist. Durch geeignete Wahl des Implantatmaterials, beispielsweise Polymethylmethacrylat, kann die Thermoplastizität des Materials in vorteilhafter Weise zur Abdichtung des Markraumes bzw. der Markhöhle ausgenutzt werden. Bei einem zylindrischen oder kegelstumpfförmigen Markraumsperrer, der die Aufgabe hat, die Markhöhle auf der distalen Seite der Prothesenspitze dicht abzuschließen, ist es besonders vorteilhaft, daß die proximale Fläche, d.h. das Dach des Markraumsperrers halbkugelförmig konvex geformt ist oder daß mindestens über einem Teilbereich der proximalen Fläche, 25 % bis 75 %, vorzugsweise etwa 50 % von deren Durchmesser, um deren Zentrum herum, eine halbkugelige Erhebung ausgebildet ist. Die Basis der Erhebung an der Dachfläche des Markraumsperrers hat typischerweise einen Durchmesser von etwa 5 mm. Auf diese Weise ist es möglich, mit einem geeigneten Applikator das Material im Zustand der plastischen Verformbarkeit so zu verformen, daß der Querschnitt des Markraumsperrers vorzugsweise in der Richtung erweitert wird, wo Spalte zwischen dem Knochen und dem Zylinderimplantat vorhanden sind. Dies ist in diesem Bereich der Markhöhle von besonderer Bedeutung, da hier die Markhöhle einen elliptischen Durchmesser mit längerer Achse in der sagittalen Ebene aufweist, so daß ein zylinderförmiger Markraumsperrer, der in frontaler Ebene die Markhöhle verschließt, in sagittaler Schnittrichtung sowohl auf der ventralen als auch auf der dorsalen Seite Spalte freiläßt. Auf diese Weise ist es möglich, sowohl bei Markraumsperrern als auch bei Markraumverschlüssen für Amputationsstümpfe auch einen ungleichmäßigen Markhöhlenquerschnitt des Knochens vollständig dicht zu verschließen. Der Applikator ist derart ausgebildet, daß seine Eingriffsfläche mit dem Implantat in ihrer Mitte eine kleine Vertiefung zum formschlässigen Erfassen der halbkugelförmigen Erhebung des Implantats aufweist und im übrigen im wesentlichen plan oder leicht konkav ausgebildet ist. Wenn ein derartiger Applikator gegen das Implantat gepreßt wird, kann durch die plastische Verformbarkeit des Materials des Implantats eine derartige Formänderung erreicht werden, daß eine vollständige Abdichtung des Markhöhlenquerschnitts möglich ist. Bei einem Verschluß eines Amputationsstumpfes kann in ähnlicher Weise eine halbkugelige Erhebung als Abdichthilfe am vorzugsweise dichten distalen Ende des Verschlusses vorgesehen sein oder das distale Ende des Verschlusses kann als Ganzes halbkugelförmig konvex geformt sein.

Die Erfindung wird nachstehend anhand der Figuren und eines Beispiels näher erläutert. Es zeigen:
- Fig. 1: eine schematische Ansicht eines erfindungsgemäßen Markraumsperrers im Maßstab 1:1,
- Fig. 2: eine Seitenansicht des Markraumsperrers gemäß Fig. 1 im Maßstab 5:1,
- Fig. 3: einen Schnitt entlang der Linie 1-1 von Fig. 2,
- die Ansicht A gemäß Fig. 2, Fig. 4
- Fig. 5: einen schematischen Teilschnitt entlang der Linie 2-2 von Fig. 4,
- Fig. 6a und 6b: schematische Ansichten des erfindungsgemäßen Markraumsperrers im Maßstab 1:1 vor und nach dem Biegen, und
- Fig. 7: eine Seitenansicht des Markraumsperrers gemäß Fig. 6b im Maßstab 5:1.

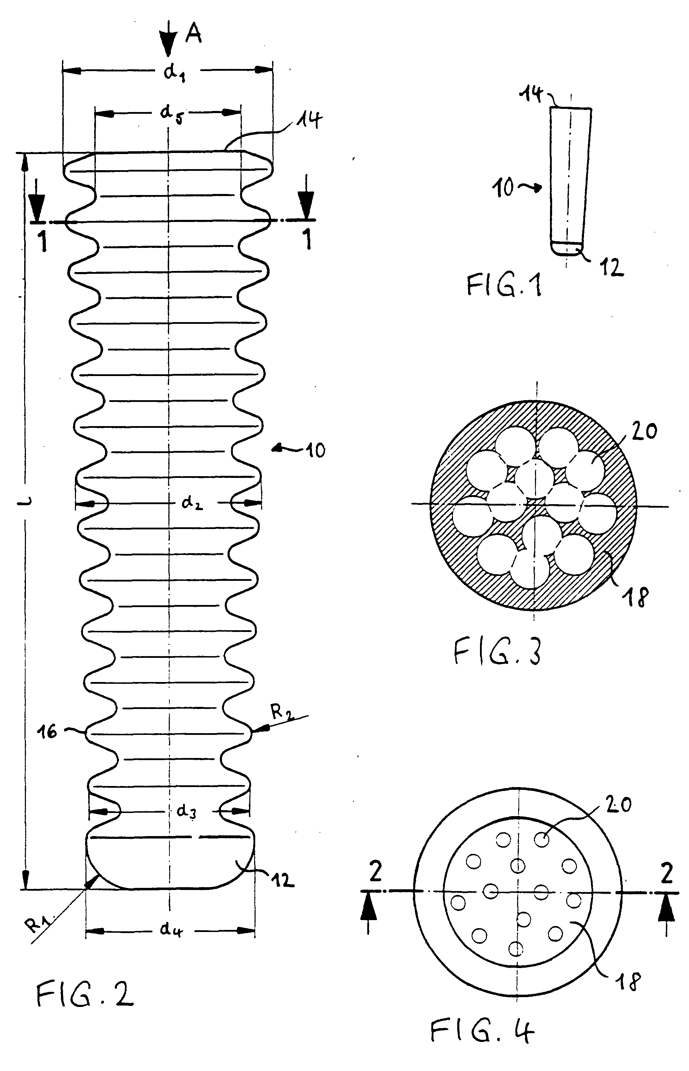

Der erfindungsgemäße Markraumsperrer 10 gemäß Fig. 1 weist die Form eines Kegelstumpfes mit einem Boden 12 und einem flachen Dach 14 auf. Der Boden 12 ist gerundet, um die Einführung des Markraumsperrers in die Markhöhle zu erleichtern.

Aus der Seitenansicht des Markraumsperrers 10 gemäß Fig. 2 ist ersichtlich, daß der kegelstumpfförmige Markraumsperrer in seinem unteren Teil in der Nähe des Bodens 12, z.B. im unteren Viertel seiner Länge, einen im wesentlichen zylindrischen Außenumfang aufweist. Die Außendurchmesser d₁, d₂, d₃ und d₄ des Markraumsperrers betragen etwa 10 mm, 9 mm, 7,5 mm bzw. 8 mm. Die Länge 1 des Markraumsperrers beträgt etwa 35 mm. Der Radius R₁ der Rundung des Bodens 12 beträgt etwa 2,5 mm.

Der Markraumsperrer weist an seinem Außenumfang mehrere Lamellen 16 mit dazwischen liegenden Vertiefungen oder Einschnitten auf. Die Lamellen dienen der Abdichtung des Markraumsperrers im Markkanal. Der Abstand der Lamellen 16 voneinander in Axialrichtung des Markraumsperrers beträgt etwa 2,5 mm. Der Innnendurchmesser d₅ am Dach beträgt etwa 7 mm, und die Tiefe der Einschnitte zwischen den einzelnen Lamellen 16 am Außenumfang des Markraumsperrers 10 beträgt somit etwa 1,5 mm. Der Lamellenradius R₂ beträgt 0,3 bis 0,5 mm.

Im Schnitt entlang der Linie 1-1 von Fig. 2 gemäß Fig. 3 ist der innere Aufbau des Markraumsperrers erkennbar. Das das Implantat bildende dreidimensionale Gerüst 18 ist schraffiert eingezeichnet und dazwischen sind die etwa kugelförmigen Poren 20 erkennbar. Die Poren 20 sind durch Herauslösen von als Platzhalter verwendeten kugelförmigen Formkörpern entstanden.

In der Ansicht gemäß Fig. 4, die eine Draufsicht des Markraumsperrers 10 gemäß Fig. 2 darstellt, sind ebenfalls die Poren 20 in dem Material des dreidimensionalen Gerüsts 18 erkennbar.

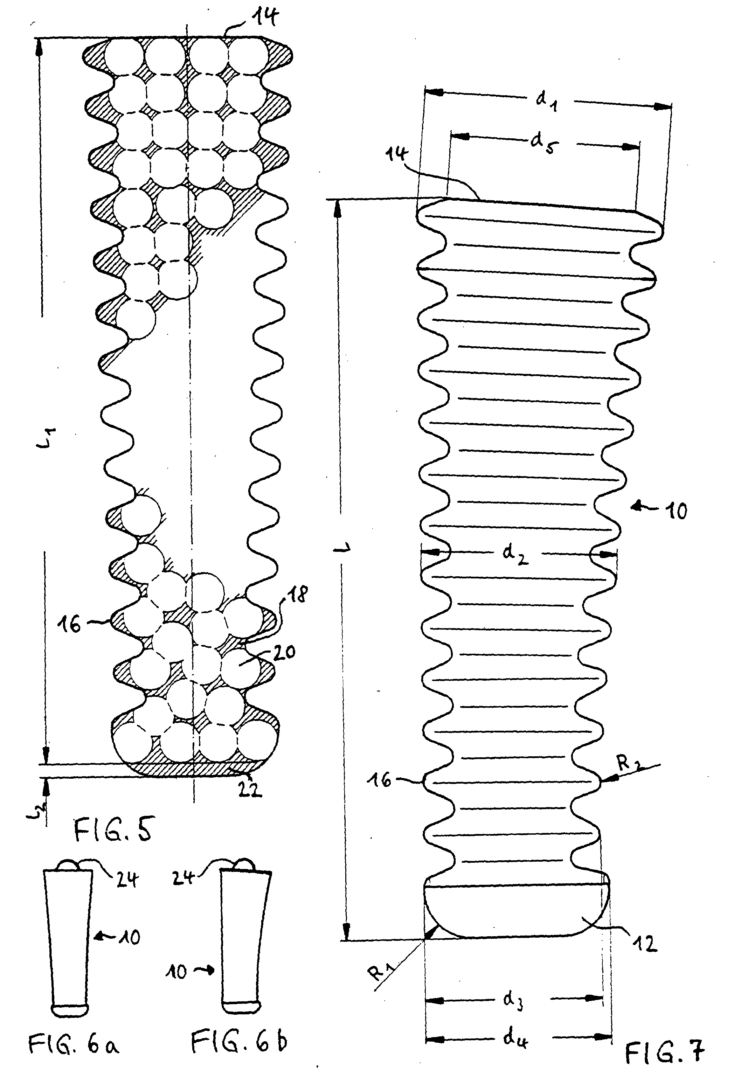

In der Schnittansicht gemäß Fig. 5 ist die Porenstruktur des erfindungsgemäßen Markraumsperrers noch deutlicher erkennbar. Der Durchmesser der Poren 20 beträgt in diesem Fall etwa 1,9 mm.

Während die Porenstruktur mit einer offenporigen Oberfläche im Bereich über die Länge l₁ des Markraumsperrers vorliegt, ist im Bereich der Länge l₂ am Boden des Markraumsperrers ein bei der Herstellung wie vorstehend beschrieben ausgebildeter Deckel 22 mit geschlossener Oberfläche vorgesehen, der einen dichten Abschluß des distalen Endes des Markraumsperrers beim Einbringen in die Markhöhle bildet. Die Länge l₂ des Deckels 22 beträgt etwa 0,5 mm.

In den Figuren 6a und 6b ist schematisch dargestellt, daß sich der Markraumsperrer 10, der in Fig. 6a im Ruhezustand dargestellt ist, thermoplastisch biegen läßt, wie in Fig. 6b dargestellt. Dies ermöglicht u.a. eine Anpassung des Markraumsperrers an die individuelle Form der Markhöhle. Der Markraumsperrer 10 gemäß Figuren 6a und 6b weist eine halbkugelförmige Erhebung 24 als Abdichthilfe auf.

In Fig. 7 ist eine Seitenansicht des gebogenen Markraumsperrers gemäß Fig. 6b (ohne halbkugelförmige Erhebung) im Maßstab 5:1 dargestellt. Bei dieser Ausführungsform beträgt die Länge 1 des Markraumsperrers 35 mm, die Durchmesser d₁, d₂, d₃ und d₄ betragen 12 mm, 9,5 mm, 8,5 mm bzw. 9 mm, und der Innendurchmesser d₅ beträgt 9 mm.

Um eine Anpassung des Markraumsperrers an die individuelle Form der Markhöhle zu gewährleisten, sind verschiedene Größen des Markraumsperrers vorgesehen. Hierbei sind folgende Abmessungen bevorzugt:
1: 35 mm
d₁ (Außendurchmesser am Deckel des Markraumsperrers): 8 bis 14 mm
d₂ (Außendurchmesser im mittleren Bereich des Markraumsperrers): 7 bis 10,5 mm
d₃ (Außendurchmesser der dem Boden des Markraumsperrers am nächsten liegenden Lamelle): 5,5 bis 9,5 mm
d₄ (Außendurchmesser des Bodens des Markraumsperrers): 6 bis 10 mm
d₅ (Innendurchmesser am Deckel des Markraumsperrers): 5 bis 11 mm
R₁: 2,5 mm
R₂: 0,3 bis 0,5 mm.

### Beispiel

In eine als stumpfer Kegelkonus mit einem Durchmesser zwischen 6 und 12 mm und einer Länge von etwa 35 mm ausgebildete Form werden Zuckerkugeln mit einem Durchmesser von 1 bis 1,5 mm lose aufgeschüttet. Anschließend wird die Form unter Anwendung von Vakuum mit Wasserdampf durchströmt, was zu einer Verklebung der Zuckerkugeln führt. Nach der anschließenden Trocknung wird die Form mit dem aus den Zuckerkugeln gebildeten Körper im Gießverfahren mit einem Gemisch aus 20 % Hydroxylapatit und 80 % PMMA ausgegossen. Nach Aushärtung des Verbundwerkstoffes PMMA/Hydroxylapatit wird der entstandene Verbundkörper aus der Form herausgenommen und in einer Drehbank mechanisch nachbearbeitet in der Weise, daß mehrere Lamellen an der Außenseite des Verbundkörpers ausgebildet werden. Nach der mechanischen Bearbeitung wird der Verbundkörper in einer Waschmaschine von seinem Zuckerinlet befreit, so daß lediglich das poröse Implantat verbleibt. Danach kann das entstandene Implantat sterilisiert werden, beispielsweise mit Ethylenoxid oder durch Strahlung. Das entstandene Implantat kann während einer Operation als Markraumstopper oder Markraumsperrer in die Markhöhle versenkt werden.

## Patentansprüche

1. Implantat, bestehend aus einem dreidimensionalen Gerüst aus einem körperverträglichen Polymer oder Kunststoff, wobei das Gerüst aus bälkchenartigen Stukturen besteht, die miteinander in Verbindung stehende und vorgebbar einstellbare Hohlräume umschließen.

2. Implantat nach Anspruch 1, dadurch herstellbar, daß die Strukturen um als Platzhalter für die Hohlräume dienende Formkörper ausgebildet werden.

3. Implantat nach Anspruch 2, wobei die Formkörper derart ausgebildet sind, daß sie zunächst miteinander verbindbar sind und nach dem Ausbilden der das Gerüst bildenden Strukturen ohne Angreifen der Strukturen entfernbar sind.

4. Implantat nach Anspruch 3, wobei die Formkörper chemisch oder physikalisch miteinander verbindbar sind und nach dem Ausbilden der Strukturen chemisch oder physikalisch entfernbar sind.

5. Implantat nach einem der Ansprüche 1 bis 4, wobei das dreidimensionale Gerüst aus einem Polymer auf der Basis eines Acrylates oder eines Polymethacrylates, einem Copolymer eines Acrylates und Methacrylates oder aus einer Mischung hieraus oder aus einem anderen körperverträglichen, resorbierbaren oder nicht resorbierbaren Kunststoff oder aus einer Polyaminosäure, einem Polylactat, einem Polyglykolat oder einem Gemisch aus verschiedenen Polyaminosäuren besteht.

6. Implantat nach einem der Ansprüche 1 bis 5, wobei das Material des dreidimensionalen Gerüsts gießbar oder spritzbar ist, beispielsweise im Spritzgußverfahren verarbeitbar.

7. Implantat nach einem der Ansprüche 2 bis 6, wobei die Formkörper aus einem leicht schmelzbaren Material, wie Wachs, oder aus Zucker oder einem anderen löslichen, vorzugsweise wasserlöslichen Material bestehen, oder wobei die Formkörper aus einem keramischen Werkstoff bestehen, wie Tricalciumphosphat oder Hydroxylapatit oder einem Gemisch aus beiden oder aus einer anderen, leicht oder schwer resorbierbaren Calciumverbindung.

8. Implantat nach einem der Ansprüche 2 bis 7, wobei die Formkörper kugelförmig sind.

9. Implantat nach einem der Ansprüche 2 bis 8, wobei die Formkörper eine Größe zwischen 0,2 bis 5 mm, vorzugsweise 1 bis 3 mm aufweisen.

10. Implantat nach einem der Ansprüche 2 bis 9, wobei eine Mischung von Formkörpern verschiedener Größe als Platzhalter verwendet wird.

11. Implantat nach einem der Ansprüche 2 bis 9, wobei die Dichte der Schüttung der Formkörper variierbar ist.

12. Implantat nach einem der Ansprüche 1 bis 11, wobei das Gerüst 0,01 bis 10 % eines Wirkstoffes enthält, der aus dem Implantat protrahiert freisetzbar ist.

13. Implantat nach Anspruch 12, wobei der Wirkstoff ein pharmazeutischer Wirkstoff ist.

14. Implantat nach einem der Ansprüche 1 bis 13, wobei das Implantat eine äußere Konfiguration in Form von Lamellen aufweist, die einen Abstand von 0,5 bis 3,0 mm voneinander haben und eine Lamellendicke von 0,3 bis 1 mm aufweisen, wobei die Lamellen gleichförmig ringförmig um das Implantat oder in Form eines Gewindes spiralförmig angeordnet sind, vorzugsweise mit unterschiedlicher Steigung zwischen 30 und 87°.

15. Implantat nach einem der Ansprüche 1 bis 14, wobei das Implantat derart ausgebildet ist, daß es zur stabilen Fixation von Verbundosteosynthesen verwendbar ist.

16. Verfahren zum Herstellen eines Implantats, insbesondere nach einem der Ansprüche 1 bis 15, mit den folgenden Verfahrensschritten:
- Verbinden von Formkörpern zu einem dreidimensionalen Formkörperkonglomerat,
- Formen eines sich vom Material der Formkörper unterscheidenden körperverträglichen Polymermaterials um die Formkörper herum zur Ausbildung eines dreidimensionalen Gerüsts, und
- Entfernen der Formkörper, so daß lediglich das dreidimensionale Gerüst aus dem Polymermaterial verbleibt und das Implantat bildet.

17. Verfahren nach Anspruch 16, wobei die Formkörper chemisch oder physikalisch miteinander verschweißt oder fest miteinander verklebt werden, beispielsweise in Wasserdampf oder im Sinterverfahren.

18. Implantat nach einem der Ansprüche 1 bis 4 oder 6 bis 17, wobei das dreidimensionale Gerüst aus einem Acrylat, einem Methacrylat oder einem Copolymer eines Acrylates und Polyurethacrylates, oder einer Mischung aus diesen ausgebildet wird.

## Claims

1. An implant comprising a three-dimensional frame formed by a polymer or plastic being compatible with the body, wherein said frame being comprised of beam-like structures which enclose interconnected and selectively adjustable cavities.

2. The implant according to claim, characterised by being producable by forming said structures around moulded bodies which act as placeables for such cavities.

3. The implant according to claim 2, wherein said moulded bodies being formed such that they can first be interconnected and thereafter be removed after said structures forming said frame have been formed without acting upon said structures.

4. The implant according to claim 3, wherein said moulded bodies may be chemically or physically interconnected and may be chemically or physically removable after said structures have been formed.

5. The implant according to claims 1 to 4, wherein said three-dimensional frame being comprised of a polymer based on an acrylate, or a polymethacrylate, a copolymer of an acrylate or methacrylate, a mixture thereof, or another resorbable or non-resorbable plastic compatible with the body, or a polyamine acid, a polyacrylate, a polyglycolate or a mixture comprising several polyamine acids.

6. The implant according to claims 1 to 5, wherein the material of said three-dimensional frame being pourable or injectable, for example by injection moulding.

7. The implant according to claims 2 to 6, wherein said moulded bodies being comprised of a readily meltable material like wax or sugar or any other soluble, preferably watersoluble material, or wherein said moulded bodies being comprised of a ceramic material, like tribasic calcium phosphate or hydroxylapatite or a mixture of both or any other calcium compound being easy or difficult to resorb.

8. The implant according to claims 2 to 7, wherein said moulded bodies being spherical.

9. The implant according to claims 2 to 8, wherein the size of said moulded bodies is between 0.2 and 5 mm, preferably between 1 to 3 mm.

10. The implant according to claims 2 to 9, wherein a mixture of moulded bodies of different sizes being used as placeables.

11. The implant according to claims 2 to 9, wherein the bulk density of said moulded bodies being variable.

12. The implant according to claims 1 to 11, wherein said frame includes 0.01 to 10% of an active substance which may be released in a protracted manner out of said implant.

13. The implant according to claim 12, wherein said active substance being a pharmaceutical substance.

14. The implant according to claims 1 to 13, wherein said implant comprising an outer configuration being formed as blades, which are spaced about 0.5 to 3.00 mm apart, having a thickness of 0.3 to 1 mm, wherein said blades being uniformly positioned around said implant in an annular fashion, or spirally as a thread, preferably having a differentiated gradient between 30 and 87°.

15. The implant according to claims 1 to 14, wherein said implant being formed such that it may be used for secure fixation of compound osteosynthesis.

16. A method for producing an implant, in particular according to claims 1 to 15, wherein said method comprising the following steps:
- Connecting moulded bodies as a three-dimensional conglomerate of moulded bodies;
- Forming a polymer being different from the material of said moulded bodies, which is compatible with the human body and positioned around such to form a three-dimensional frame; and
- Removing said moulded bodies, such that there only remains said three-dimensional frame of said polymer, building said implant.

17. The method according to claim 16, wherein said moulded bodies being chemically or physically welted or securely bonded together, as for example by water vapour or a sinter process.

18. The implant according to claims 1 to 4 or claims 6 to 17, wherein said three-dimensional frame being formed from an acrylate, a methacrylate or a copolymer of an acrylate and polymethacrylates or a mixture thereof.

## Revendications

1. Implant constitué par une charpente tridimensionnelle en polymère ou en matière plastique bien tolérée par l'organisme, la charpente étant composée de structures formant des sortes de petites barres reliées entre elles et renfermant des cavités qui peuvent être prédélimitées.

2. Implant selon revendication 1, réalisable compte tenu du fait que les structures sont configurées de sorte à servir d'écarteur pour les corps moulants qui définissent les cavités.

3. Implant selon revendication 2, les corps moulants étant configurés de telle sorte qu'ils peuvent être tout d'abord reliés entre eux et, après la configuration des structures qui constituent la charpente, ils peuvent ensuite être retirés sans porter atteinte aux structures.

4. Implant selon revendication 3, les corps moulants pouvant être reliés par voie chimique ou physique, puis retirés par voie chimique ou physique, après configuration des structures.

5. Implant selon l'une des revendications 1 à 4, la charpente tridimensionnelle étant réalisée en un polymère sur la base d'un acrylate ou d'un polyméthacrylate, d'un copolymère, d'un acrylate ou d'un méthacrylate, ou bien d'un mélange qui en est tiré, ou encore d'une autre matière plastique bien tolérée par l'organisme, résorbable ou non, ou d'un acide polyaminé constitué d'un polylactate, d'un polyglycolate ou d'un mélange de ces divers acides polyaminés.

6. Implant selon l'une des revendications 1 à 5, la matière qui compose la charpente tridimensionnelle pouvant être travaillée par coulage ou par injection, par exemple à l'aide du procédé de moulage par injection.

7. Implant selon l'une des revendications 2 à 6, les corps moulants étant faits d'une matière facilement liquéfiable telle que la cire ou le sucre, ou encore d'une autre matière de préférence hydrosoluble, ou bien les corps moulants étant constitués par une céramique, une matière telle que du phosphate tricalcique ou par de l'hydroxylapatite ou un mélange des deux, ou bien encore par une autre combinaison avec le calcium difficilement résorbable.

8. Implant selon l'une des revendications 2 à 7, les corps moulants étant de forme globulaire.

9. Implant selon l'une des revendications 2 à 8, les corps moulants présentant une grandeur de 0,2 à 5 mm, de préférence de 1 à 3 mm.

10. Implant selon l'une des revendications 2 à 9, un mélange de corps moulants de différentes grandeurs étant utilisé en tant qu'écarteur.

11. Implant selon l'une des revendications 2 à 9, la densité du tas des corps moulants pouvant être renndue variable.

12. Implant selon l'une des revendications 1 à 11, la charpente renfermant une matière, à raison de 0,01 à 10 %, qui peut être libérée de l'implant par passage progressif.

13. Implant selon la revendication 12, la matière active étant une matière pharmaceutique.

14. Implant selon l'une des revendications 1 à 13, l'implant présentant une configuration externe sous forme de lamelles séparées entre elles par une distance de 0,5 à 3,0 mm et présentant une densité des lamelles de 0,3 à 1 mm, les lamelles étant de forme semblable et régulièrement disposées en anneau autour de l'implant ou sous forme d'un filetage, en spirale, de préférence avec une pente variant entre 30 et 87°.

15. Implant selon l'une des revendications 1 à 14, l'implant étant configuré de sorte à pouvoir servir de fixation stable pour les ostéosynthèses de fusion.

16. Procédé de fabrication d'un implant, en particulier selon l'une des revendications 1 à 15, avec les étapes de procédé suivantes :
- liaison des corps moulants en conglomérat de corps moulants tridimensionnel
- moulage d'une matière en polymère, bien tolérée par l'organisme et différente de la matière des corps moulants, autour des corps moulants pour constituer une charpente tridimensionnelle et
- enlèvement des corps moulants de sorte à ce que seule la charpente tridimensionnelle en polymère reste en place et constitue l'implant.

17. Procédé selon revendication 16, les corps moulants étant soudés entre eux par voie chimique ou physique, ou solidement collés entre eux, par exemple en présence de vapeur d'eau ou par un procédé de frittage.

18. Implant selon l'une des revendications 1 à 4 ou 6 à 17, la charpente tridimensionnelle étant configurée en un acrylate, un méthacrylate ou en un copolymère d'acrylate et de polyméthacrylate ou encore en un mélange des deux.
